(19) Europäisches Patentamt

European Patent Office

Office européen des brevets

(11) Numéro de publication : **0 385 902 B1**

(12) **FASCICULE DE BREVET EUROPEEN**

(45) Date de publication du fascicule du brevet :
**29.07.92 Bulletin 92/31**

(21) Numéro de dépôt : **90460010.3**

(22) Date de dépôt : **01.03.90**

(51) Int. Cl.⁵ : **B65B 43/44,** C12M 1/22

---

(54) **Dispositif pour remplir des boîtes de Pétri.**

---

(30) Priorité : **03.03.89 FR 8903008**

(43) Date de publication de la demande :
**05.09.90 Bulletin 90/36**

(45) Mention de la délivrance du brevet :
**29.07.92 Bulletin 92/31**

(84) Etats contractants désignés :
**DE GB**

(56) Documents cités :
**GB-A- 2 018 288**
**US-A- 2 987 163**

(72) Inventeur : **Borie,Gérard**
**3 Domaine du Landry**
**F-35000 Rennes (FR)**
Inventeur : **Le Roch,Alain**
**3 rue Pierre Loti**
**F-22600 Loudéac (FR)**
Inventeur : **Pellegrin,Jean**
**3 rue Frédéric Mistral**
**F-32000 Rennes (FR)**

(74) Mandataire : **Le Faou, Daniel et al**
**Cabinet Regimbeau 11, rue Franz Heller,**
**Centre d'Affaires Patton B.P. 19107**
**F-35019 Rennes Cédex (FR)**

(73) Titulaire : **ARMOR EQUIPEMENT**
**SCIENTIFIQUE**
**Route de Dol**
**F-35270 Combourg (FR)**

## Description

La présente invention concerne un dispositif pour remplir des boîtes de Pétri. Les boîtes de Pétri sont de petites boîtes de contour circulaire, en matière plastique transparente, qui contiennent un produit nutritif tel qu'un mélange de gélose et d'eau distillée; elles servent de milieu de développement pour des cultures contenant des micro-organismes et sont utilisées en grand nombre par les laboratoires d'analyse notamment dans la recherche médicale et dans l'industrie.

Le mélange gélose/eau distillée se présente à température ordinaire sous une forme solide, ayant la consistance d'une gélatine. Avant d'être introduit dans les boîtes à une température de l'ordre de 42 à 50° C, le mélange est porté à une température de l'ordre de 100° C pour prendre une forme liquide ; il se répartit ainsi dans les boîtes pour former une couche régulière, à surface plane. La boîte peut être utilisée seulement après durcissement du produit, la durée de durcissement étant de l'ordre de dix minutes à température ambiante.

Il a déjà été proposé des dispositifs destinés à effectuer le remplissage automatique des boîtes.

Ainsi, dans le document FR-A-2 433 752, est décrit un dispositif de remplissage dans lequel les boîtes à remplir sont acheminées les unes après les autres au moyen d'un plateau horizontal tournant, à un poste de remplissage qui comporte une buse de distribution de gélose. Les boîtes se présentent au poste de distribution de gélose à l'état ouvert, c'est-à-dire couvercle maintenu écarté au-dessus du fond, les unes après les autres. Ceci est possible grâce au fait que le plateau ce transfert tournant est composé de deux platines horizontales circulaires coaxiales et superposées, qui sont percées chacune d'ouvertures circulaires régulièrement réparties à leur périphérie et en correspondance l'une avec l'autre ; les ouvertures de la platine supérieure ont un diamètre légèrement plus petit que celui des couvercles mais légèrement plus grand que celui des fonds de boîtes, de sorte qu'elles autorisent le passage de ces derniers en retenant seulement les couvercles ; au contraire, la platine inférieure est pourvue d'ouvertures conformées pour recevoir et supporter les fonds au cours de leur transfert.

La buse distributrice de gélose s'étend entre les deux platines, ce qui permet d'approvisionner en gélose les fonds de boîtes se présentant tour-à-tour à la station de remplissage.

Ce dispositif connu est équipé d'un carrousel unique qui fait à la fois office de station distributrice de boîtes vides et de station réceptrice des boîtes pleines; le carrousel contient plusieurs piles de boîtes qui sont réparties à sa périphérie en formant des colonnes, ces dernières étant retenues par des tiges verticales.

Ce dispositif connu donne satisfaction de manière générale mais présente toutefois certains inconvénients.

En premier lieu, la capacité de contenance en boîtes du dispositif, et partant, son autonomie, sont limitées du fait de l'utilisation d'un carrousel unique ; un accroissement du nombre de colonnes par carrousel poserait des difficultés liées au poids et à l'encombrement de celui-ci.

En second lieu, le système de transfert des boîtes depuis le carrousel au plateau de transfert, et inversement de celui-ci au carrousel (après remplissage) est relativement compliqué et sujet à des pannes car il nécessite l'utilisation de moyens mobiles qui assurent la retenue de l'avant-dernière boîte de la colonne lors du prélèvement ou de l'introduction de la dernière, c'est-à-dire de la boîte la plus basse; la complexité de ce système nécessite par ailleurs de travailler à une cadence relativement faible.

En troisième lieu, si, comme cela est relativement fréquent, il apparaît des défauts dans le processus de remplissage, ces défauts pouvant conduire à des éclaboussures ou des déversements du produit à côté des boîtes, il se pose un problème lié à la difficulté de nettoyer le dispositif; en effet le nettoyage nécessite de nombreux démontages, notamment celui du plateau, et entraîne une immobilisation relativement longue du dispositif, immobilisation durant laquelle la production est arrêtée.

En quatrième lieu, l'empilage des boîtes pleines se fait alors que le produit est encore liquide, ce qui est une cause de défaut de planéité de surface de la gélose dans les boîtes, ceci en raison de l'agitation inévitable du produit qui apparaît durant le stockage ; hors, un défaut de planéité risque de poser des difficultés notamment au cours des analyses ultérieures faites par lecture à l'aide d'une caméra.

Enfin, il n'est pas possible de prélever pour utilisation immédiate une colonne de boîtes pleines venant d'être stockées dans le carrousel en raison du temps de refroidissement nécessaire du produit, comme cela a déjà été mentionné plus haut ; ceci peut être un inconvénient lorsque le personnel utilisateur se trouve à cours de boîtes de Pétri et en a un besoin d'utilisation urgent.

L'invention vise à résoudre ces différents problèmes en proposant un dispositif du genre évoqué qui soit de conception simple et d'un fonctionnement sûr et fiable, qui travaille à cadence élevée, qui soit d'un entretien facile notamment en vue du nettoyage, qui ait une capacité de stockage élevée, c'est-à-dire qui puisse contenir et traiter sans intervention extérieure un très grand nombre de boîtes, qui permette d'obtenir ces couches de gélose à surface bien plane, et qui produise des boîtes de Pétri immédiatement utilisables (sans délai d'attente). Ces différents résultats sont atteints conformément à l'invention, grâce au fait que le dispositif qui - comme le dispositif déjà connu mentionné plus haut - comporte un plateau de transfert tournant, apte à acheminer successivement et pas-à-pas des boîtes ouvertes (couvercles maintenus écartés au-dessus du fond) d'une station distributrice de boîtes vides à une station de remplissage, puis de cette dernière à une station réceptrice de boîtes remplies, ce plateau étant composé de deux platines horizontales circulaires coaxiales et superposées, percées chacune d'une série d'ouvertures circulaires régulièrement réparties à leur périphérie et en correspondance l'une avec l'autre, les ouvertures de la platine supérieure ayant un diamètre légèrement plus petit que celui des couvercles mais légèrement plus grand que celui des fonds, de sorte qu'elles autorisent le passage de ces derniers en retenant seulement les couvercles, est caractérisé en ce que :

– d'une part, les ouvertures de la platine inférieure ont un diamètre légèrement plus grand que celui des fonds de telle sorte qu'elles autorisent également le passage de ces derniers ;

– d'autre part, ledit plateau de transfert repose, par sa platine inférieure sur une semelle fixe plane et horizontale, de telle manière que durant leur transfert au moyen de ladite platine inférieure, les fonds de boîte se trouvent en appui sur ladite semelle, l'entraînement du plateau en rotation étant réalisé par des moyens moteurs situés sous cette semelle, par l'intermédiaire d'un accouplement magnétique.

Par ailleurs, selon d'autres caractéristiques intéressantes de l'invention :

– ladite semelle est réfrigérée, par exemple au moyen de modules de refroidissement à effet Peltier ;

– lesdites stations distributrice et réceptrice comprennent des moyens de stockage distincts constitués par des carrousels identiques dans lesquels les boîtes sont empilées en colonnes, la rotation pas-à-pas de ces carrousels se faisant en synchronisme avec celle dudit plateau de transfert de sorte que les boîtes vides quittent une à une le carrousel distributeur en s'échappant successivement du bas des colonnes voisines tandis que les boîtes remplies sont introduites une à une dans le carrousel récepteur par la base de colonnes voisines ;

– les colonnes de boîtes contenues dans lesdits carrousels reposent sur une plaque horizontale recouvrant ledit plateau de transfert, cette plaque étant traversée, à chacune des stations distributrice et réceptrice, d'une ouverture qui autorise le passage des boîtes du carrousel distributeur au plateau, et de ce dernier au carroussel récepteur ;

– les moyens d'entraînement en rotation desdits carrousels impriment à ceux-ci, pour chaque boîte, deux demi-courses successives correspondant à la moitié de l'écart angulaire entre deux colonnes voisines ;

– il est pourvu de deux pistons de transfert situés l'un à la station distributrice, et l'autre à la station réceptrice et dont le diamètre est sensiblement plus petit que celui des boîtes, des moyens - tels que des cames rotatives - étant prévus pour déplacer verticalement ces pistons dans l'axe de l'ouverture associée ;

– lesdits moyens permettent d'amener successivement le piston dans chacune des positions suivantes :

a) une position dite haute, dans laquelle sa face supérieure se trouve sensiblement à fleur de ladite plaque horizontale ;

b) une position dite intermédiaire, dans laquelle sa face supérieure se trouve à un niveau inférieur de celui de ladite plaque horizontale, d'une hauteur correspondant sensiblement à celle d'une boîte ;

c) une position dite basse, dans laquelle sa face supérieure se trouve sensiblement à fleur de ladite semelle ;

– la face supérieure du piston présente un pan biseauté et/ou un chanfrein ;

– l'entraînement du plateau de transfert est réalisé au moyen d'un disque moteur horizontal disposé juste en-dessous de la semelle, à l'aplomb du plateau, ce disque et la platine inférieure étant pourvus chacun d'une série d'aimants régulièrement répartis à leur périphérie en vis-à-vis les uns des autres ;

– ladite platine inférieure prend appui sur la semelle par l'intermédiaire de moyens antifriction tels que des billes et/ou des roulements à billes ;

– ladite station de remplissage comprend une buse d'injection de produit tel que de la gélose, qui est alimentée par une pompe, et est normalement positionnée horizontalement entre les deux platines du plateau de transfert, et que cette buse est portée par un levier pouvant être escamoté par basculement pour dégager l'accès de la semelle et autoriser l'enlèvement dudit plateau ;

– ladite platine inférieure est échancrée vers l'extérieur au niveau de chaque ouverture, de manière à permettre une détection de la présence d'un fond de boîte dans cette ouverture au moyen d'un détecteur opto-électronique situé à la station de remplissage ;

– il est équipé d'un dispositif germicide comprenant un tube de rayonnement ultraviolet, auquel sont exposées les boîtes au cours de leur passage à la station de remplissage.

L'invention sera mieux comprise par la description et les dessins annexés qui en présentent un mode de réalisation préférentiel.

Sur ces dessins :

– la figure 1 est une vue de dessus schématique, avec arrachements partiels du dispositif ;
– la figure 2 représente à plus grande échelle le dispositif de la figure 1 coupé par le plan vertical brisé II-II ;
– la figure 3 représente schématiquement et incomplètement en perspective un carroussel de stockage de boîtes ;
– les figures 4, 5, 6 et 7 sont des vues schématiques, en coupe verticale, représentant les différentes étapes de prélèvement et de transfert de boîtes vides jusqu'à la station de remplissage ;
– les figures 4a et 5a sont des vues de dessus schématiques (à plus petite échelle) correspondant respectivement aux situations illustrées aux figures 4 et 5 ;
– les figures 8 et 9 sont des vues schématiques en coupe verticale illustrant le principe de stockage d'une boîte pleine à la station réceptrice ;
– la figure 10 est une vue de côté schématique de la machine, destinée à montrer de quelle manière se fait l'enlèvement du plateau de transfert, notamment en vue du nettoyage du dispositif.

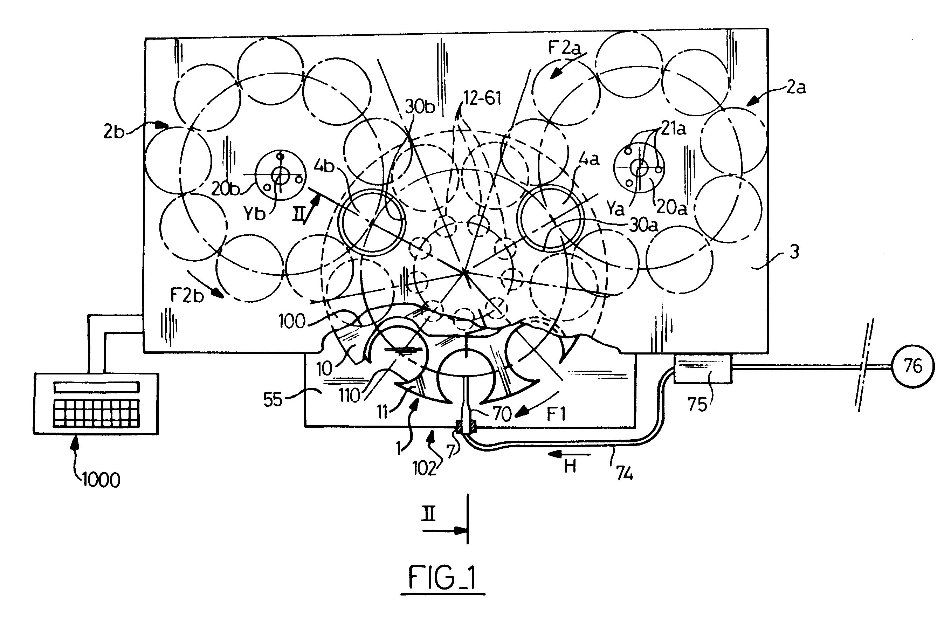

Le dispositif représenté schématiquement en vue de dessus à la figure 1 a la forme générale d'un caisson rectangulaire recouvert par une plaque 3 en acier inoxydable. A l'intérieur du caisson sont installés les différents équipements de pilotage du dispositif, en particulier les moteurs d'entraînement des pièces en mouvement et les composants électroniques constitutifs du micro-processeur pilotant la machine; ce dernier est connecté à un clavier 1000 qui de manière connue permet à l'opérateur de répondre aux demandes d'instructions du micro-processeur et de lui donner des consignes en fonction de l'opération qu'il souhaite réaliser ; ces consignes visent notamment le nombre de boîtes à traiter et le volume de gélose qui doit être distribué dans chaque boîte.

On a désigné par la référence 102 la face avant du dispositif, dirigée vers le bas de la figure 1, face qui est tournée vers l'opérateur. Dans la partie centrale du dispositif, à proximité de cette face avant, est monté un plateau rotatif 1. Vers l'arrière du dispositif, symétriquement de chaque côté du plateau 1, sont situées les stations distributrices de boîtes vides et réceptrices de boîtes pleines. Les éléments constitutifs de ces deux stations sont identiques et ont par conséquent reçu les mêmes références, l'indice (a) étant attribué aux éléments équipant la station distributrice (qui se trouve à la droite de la figure 1) et l'indice (b) aux éléments de la station réceptrice (qui se trouve sur la gauche).

La station de remplissage des boîtes en produit nutritif, tel qu'un mélange de gélose et d'eau distillée, située sur la face avant du dispositif dans la partie centrale de celui-ci, comprend une buse ou canule de distribution 70 qui est réliée par un conduit flexible 74 a une source 76, en l'occurence un récipient contenant la gélose à l'état liquide. Une pompe appropriée 75, de préférence du type péristaltique, est adaptée pour pomper à la source 76, sans à-coups (afin de ne pas générer de bulles) des volumes successifs de gélose qui sont ensuite injectés dans les boîtes par la buse 70. La flèche H symbolise le passage de la gélose dans le conduit 74. La pompe 75 peut être à une ou à deux voies et adaptée pour distribuer un volume variable, compris de préférence entre 3 et 25 ml. La cadence de remplissage est avantageusement d'environ 10 boîtes à la minute pour 15 ml de produit.

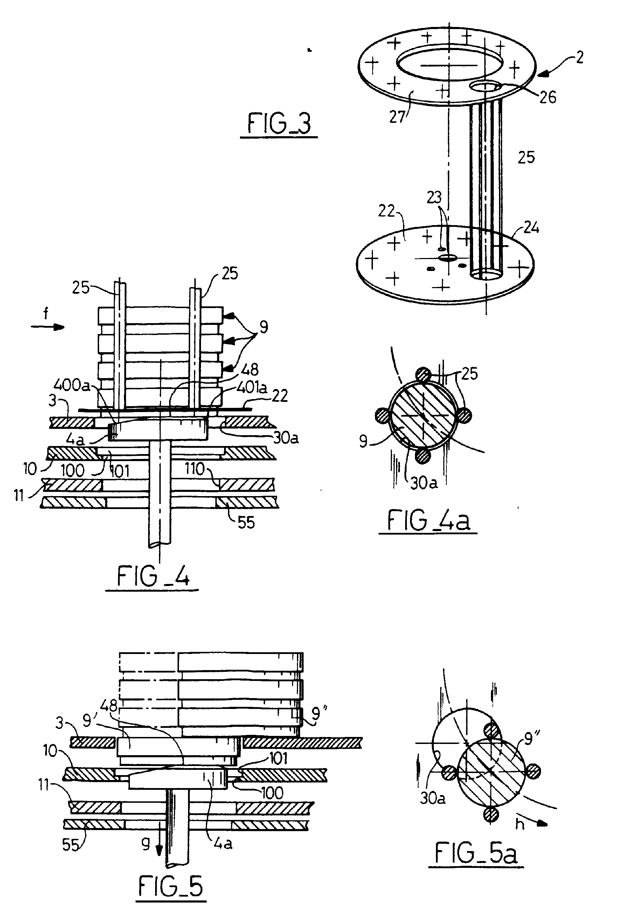

Le dispositif comprend, dans chacune des stations distributrice et réceptrice, un disque rotatif 20 d'entraînement d'un carrousel contenant les boîtes. Ce disque est monté rotatif autour d'un axe vertical Y et est entraîné par un moteur électrique approprié logé sous la plaque 3, à l'intérieur du caisson formant le bâti du dispositif. Les disques 20 possèdent des tétons de centrage et d'entraînement 21 des carrousels. Comme on le voit à la figure 3, chacun des carrousels est composé d'un disque inférieur horizontal 22 et d'une couronne supérieure 27 ; le disque 22 présente des orifices 23 appropriés pour être mis en place sur le disque 20 et coopérer avec les tétons de centrage et d'entraînement 21. Ce disque comprend une série d'ouvertures périphériques 24, angulairement réparties de manière régulière ; ces ouvertures 24 présentent un diamètre légèrement supérieur à celui des boîtes. A la figure 3 seule une ouverture 24 a été représentée ; il est prévu neuf ouvertures 24 décalées de 40° les unes par rapport aux autres. Dans la couronne supérieure 27 sont prévues des ouvertures 26 correspondantes, et les pièces 22, 27 sont reliées l'une à l'autre au moyen d'une série de tiges verticales 25 ; il est prévu quatre tiges 25 par paire d'ouvertures 24, 26, ces tiges servant à retenir un empilement de boîtes composant une colonne verticale logée entre ces tiges. Chaque colonne contient avantageusement cinquante boîtes de Pétri, de sorte que le carrousel peut contenir un total de 450 boîtes. Pour chaque ensemble de quatre tiges 25 prévues par colonne, l'une au moins de ces tiges, située vers l'extérieur du carrousel, est flexible et son extrémité supérieure est mobile, ce qui facilite l'enlèvement de la colonne de boîtes ou d'une partie des boîtes constituant cette colonne.

Les carrousels, après mise en place sur les disques 20a et 20b, sont entraînés pas-à-pas par ces derniers dans un sens qui, en vue de dessus, est le sens contraire de celui des aiguilles d'une montre, ce sens étant figuré par les flèches F2a et F2b à la figure 1.

Le plateau de transfert 1 est constitué de deux platines circulaires 10, 11 solidaires l'une de l'autre au moyen d'une série d'entretoises 13. Ils sont avantageusement réalisés dans une matière plastique à haute résistance. La platine supérieure 10 est percées d'une série de neuf ouvertures disposées à sa périphérie et régulièrement réparties tous les 40°. Chacune de ces ouvertures 100 présente un renfoncement 101 de diamètre légèrement plus grand dirigé vers le haut. La platine inférieure 11 a un diamètre légèrement plus petit que la platine 10 et présente également neuf ouvertures périphériques 110, réparties tous les 40°, dont les axes verticaux coïncident avec ceux des ouvertures 100. En raison du plus faible diamètre de la platine 11, les ouvertures 110 débouchent vers l'extérieur par des échancrures.

Traditionnellement, les boîtes de Pétri sont de petites boîtes en matière plastique transparente composées de deux parties, à savoir un fond et un couvercle. Chacune de ces parties possède une paroi latérale de diamètre différent, la paroi du couvercle venant recouvrir celle du fond lorsque la boîte est fermée ; cette mise en place se fait pratiquement sans frottement, si bien que l'enlèvement du couvercle se fait sans effort.

Selon une caractéristique importante de l'invention, l'ouverture circulaire 100 de la platine inférieure a un diamètre plus petit que celui du couvercle, de sorte que si on déplace de haut en bas une boîte dans l'axe de cette ouverture, le couvercle reste retenu par le rebord du renfoncement 101. Par contre, le diamètre de l'ouverture 100 est légèrement plus grand que celui du fond de boîte, si bien que celui-ci peut continuer à descendre en traversant l'ouverture 100 et en se libérant du couvercle ; l'ouverture circulaire 110 quant à elle possède également un diamètre très légèrement plus grand que celui du fond de boîte.

Dans la face inférieure de la platine 11 sont logés une série d'aimants 12, par exemple également au nombre de neuf régulièrement répartis angulairement tous les 40°. Le plateau 1 repose par l'intermédiaire d'une série de billes 14, par exemple trois billes disposées à 120°, qui sont avantageusement logées dans des entretoises 13, sur une plaque horizontale fixe 55, qui sera appelée semelle. Les billes pourraient être avantageusement remplacées par des roulements à billes. La plaque 55 est réalisée dans un matériau amagnétique, de préférence en alliage d'aluminium. On a désigné par la référence 5 une plaque massive, constitutive du bâti du dispositif celle-ci s'étend à faible distance sous la semelle 55 ; elle porte plusieurs paliers de guidage 50, 51, 52, d'axe vertical dont le rôle sera expliqué plus loin.

Le palier 52 sert à guider en rotation, par l'intermédiaire de roulements à billes appropriés, un arbre 60 d'axe vertical X. Ce dernier est solidaire d'un disque 6, également en matériau amagnétique tel qu'un alliage d'aluminium, qui est disposé horizontalement juste en-dessous de la semelle 55 ; le disque 6 porte une série d'aimants 61 identiques aux aimants 12 et disposés chacun en correspondance avec ces derniers. L'arbre 60 est entraîné en rotation par un moteur approprié non représenté, par exemple par l'intermédiaire d'une courroie crantée 63 engrenant sur un pignon 62 calé sur l'arbre 60. Si on place le plateau 1 sur la semelle 55 de telle sorte que l'axe du plateau soit voisin de l'axe X, par attraction magnétique mutuelle des aimants 61 et 12, le plateau se positionne exactement en alignement avec le disque, de manière à ce que les aimants 12 et 61 se trouvent exactement en vis-à-vis ; par la suite, la rotation du disque 6 réalise, par accouplement magnétique, l'entraînement correspondant du plateau 1.

A chacune des stations distributrice et réceptrice, en vis-à-vis de la trajectoire des carroussels 2a et 2b, la plaque supérieure 3 est percée d'une ouverture 30a, respectivement 30b, dont le diamètre est légèrement supérieur à celui des boîtes. Au cours de la rotation du plateau 1, après positionnement de ce dernier dans l'axe X, chaque paire d'ouvertures 100, 110 peut venir en correspondance avec ces deux ouvertures 30a, 30b.

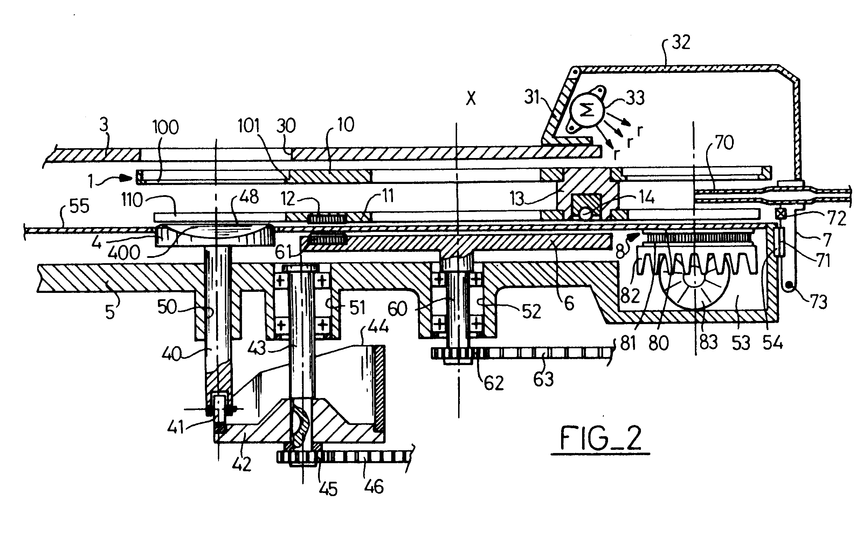

Dans le palier 50 de la plaque de bâti 5 sont guidés en translation d'axe vertical deux pistons 4a, 4b en alignement avec les ouvertures 30a, respectivement 30b. A la figure 2 est représenté le piston 4b de la station réceptrice. Un piston identique est situé à la station distributrice.

Les pistons 4 possèdent une face supérieure 48 à bord chanfreiné 401 et présentant un pan incliné 400 qui, comme on le verra plus loin, facilite le passage du fond des boîtes sur la face 48.

La tête de piston 4 est portée par une tige 40 dont l'extrémité inférieure est munie d'un galet de roulement 41 qui s'appuie contre un chemin de came annulaire 44 ; ce chemin de came est fixé sur un disque 42, celui-ci étant calé sur un arbre vertical 43 guidé en rotation dans des roulements à billes logés dans l'alésage 51. Un moto-réducteur électrique approprié (non représenté) entraîne la came 42 en rotation, par l'intermédiaire d'une courroie crantée 46 qui engrene avec un pignon 45 solidaire de l'arbre 43. La rotation de la came 42 provoque le déplacement vertical du piston. Comme on le verra par la suite, le profil de la came est déterminé de manière à donner au piston trois positions déterminées durables, une position basse, une position haute et une position intermédiaire.

Du côté de la face avant du dispositif, la plaque de bâti 5 est évidée de manière à former une boîte 53 s'étendant sous une partie de la trajectoire des boîtes lorsqu'elles sont transportées par le plateau tournant 1. Dans cette boîte 53 est disposé un dispositif réfrigérant 8. Ce dernier consiste en un module de refroidissement à effet Peltier 8, convenablement alimenté en énergie électrique par des moyens non représentés sur les figures. On sait qu'une cellule à effet Peltier se comporte comme une pompe à chaleur, sans pièces en mouvement. Elle est composée d'une face froide où l'énergie thermique est absorbée par les électrons passant d'un semi-conducteur à l'autre ; la source de courant fournit l'énergie nécessaire pour faire passer les électrons à travers le système ; la face chaude libère l'énergie à l'air ambiant à travers un échangeur de chaleur. La face froide 80 du module 8 est collée directement sous la semelle 55, tandis que la face chaude 81, dirigée vers le bas, porte des ailettes 82 de dissipation de la chaleur. Un système de ventilation comprenant un ou plusieurs ventilateurs 83 permet de faire passer un flux d'air à température ambiante dans la boîte 53 afin d'éliminer les calories dissipées par ces ailettes 82. Le module 8 réalise donc une réfrigération constante de la semelle 55.

Il peut être prévu plusieurs modules 8, avantageusement branchés en série, et disposés en arc de cercle de manière à couvrir au moins une partie de la trajectoire des boîtes, lorsque celles-ci circulent dans le dispositif au moyen du plateau tournant 1. La buse de distribution de gélose 70 est disposée horizontalement, et s'étend entre les deux platines 10 et 11, de telle manière que son orifice de sortie coïncide sensiblement avec le centre des ouvertures 100, 110. La buse 70 est emmanchée, de manière amovible, dans une gorge prévue à l'extrémité supérieure d'un levier 7 qui est articulé sur le bâti du dispositif autour d'un axe horizontal 73. Ce levier 7 est normalement maintenu en position verticale au moyen d'un aimant 54 porté par le bâti, coopérant avec un autre aimant 71 solidaire du levier. Le levier 7 peut donc être facilement basculé vers l'avant par pivotement autour de l'axe 73, ce qui permet de dégager la face avant du dispositif afin notamment de retirer le plateau 1 en vue du nettoyage, comme on le verra par la suite.

Au dessus de la station de remplissage, où se trouve la buse 70, est prévu un capot protecteur 32, de préférence en matière plastique transparente, articulé sur un support 31 solidaire de la plaque supérieure 3. Sous le capot 32 est fixé un dispositif germicide constitué par une lampe 33 émettant un rayonnement ultraviolet ; ce rayonnement, figuré par des flèches r est dirigé vers les boîtes passant au poste d'alimentation de gélose, et a pour rôle de détruire les germes microbiens et autres micro-organismes pouvant se trouver dans les boîtes, de manière à assurer une production de boîtes remplies d'une gélose parfaitement stérile.

On notera par ailleurs qu'il est prévu à la station de remplissage un détecteur représenté schématiquement à la figure 2 sous la référence 72 ; il s'agit d'un détecteur de type connu, par exemple opto-électronique à cellules émettrice et réceptrice d'un rayonnement électromagnétique, dirigé vers l'échancrure correspondant aux ouvertures 110 ménagées dans la platine inférieure. En l'absence d'un fond de boîte dans l'ouverture, le rayonnement n'est pas réfléchi, ce qui indique l'absence d'un fond, et un signal indicatif de cette anomalie est alors fourni au microprocesseur.

Le dispositif est utilisé de la manière suivante :

Un carrousel contenant le nombre de boîtes de Pétri que l'on souhaite remplir est mis en place à la station distributrice de boîtes, le disque de fond 22 du carrousel étant placé sur le disque d'entraînement 20a. Les boîtes à traiter sont disposées régulièrement dans chacune des colonnes délimitées par les tiges verticales 25, de manière à ce que toutes ces colonnes contiennent le même nombre de boîtes, à une boîte près (si le total n'est pas divisible par 9). A la station réceptrice est mis en place un autre carroussel qui lui est vide de toute boîte.

L'opérateur, à l'aide du clavier 1000, donne au microprocesseur des instructions quant à l'opération qui doit être effectuée en lui indiquant notamment le nombre de boîtes à traiter et le volume de gélose qui doit être distribué par boîte. Au début de l'opération, le disque d'entraînement 6 est mis brièvement en rotation de manière à réaliser un parfait centrage du plateau de transfert 1 dans l'axe X, pour le cas où le centrage initial ne se serait pas fait convenablement de manière automatique, et afin de rattraper les petits jeux éventuels. Les cames 44 se trouvent dans une position telle que le piston 4a se trouve en position haute, c'est-à-dire que sa face supérieure 48 se trouve à fleur de la face supérieure de la plaque 3 ; inversement le piston 4b se trouve en position basse, de telle manière que sa face supérieure 48 se trouve à fleur de la semelle 55 (position de la figure 2).

Des détecteurs appropriés sont prévus pour contrôler au départ et aussi durant toute l'opération, le bon positionnement des carrousels et du plateau de transfert.

Le transfert d'une boîte vide et son amenée à la station d'alimentation vont maintenant être décrits en référence aux figures 4 à 7. Le carroussel 2a est mis en rotation de telle manière que l'une de ses ouvertures d'échappement des boîtes 24 arrive en vis-à-vis de l'ouverture 30a ; le piston 4a se trouve en position haute (figure 4). Le mouvement d'amenée de la colonne de boîtes vides est figuré par la flèche f à la figure 4. Le pan incliné 400a favorise le passage progressif de la face inférieure du fond de boîte de la plaque 3 sur le piston 4a. La came 42a est alors mise en rotation pour provoquer la descente du piston 4a dans une position intermédiaire dans laquelle la face 48 est en retrait vers le bas d'une hauteur correspondant à celle d'une boîte ; ce mouvement de descente sur une course limitée est figuré par la flèche g à la figure 5. La boîte inférieure de la pile, référencée 9', est ainsi extraite du carrousel. Ensuite, le carrousel 2a est mis en rotation sur une course angulaire de 20°, de telle manière que la pile de boîtes restantes se trouve à cheval, par la boîte inférieure 9″, sur la plaque 3 et sur l'ouverture 30a (voir figure 5a, où ce mouvement de rotation du carrousel a été figuré par la flèche h).

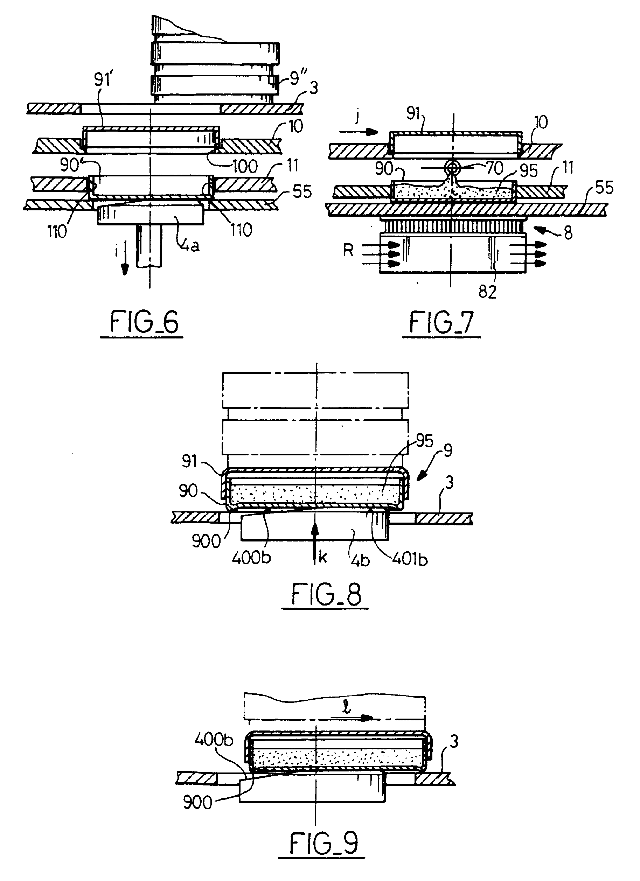

La poursuite de la rotation de la came 42 provoque alors la descente complète du piston 4a, lequel atteint une position basse où sa face supérieure 48 se trouve dans le même plan que la face supérieure de la semelle 55. Au cours de ce mouvement de descente figuré par la flèche i à la figure 6, le couvercle 91' de la boîte 9' est retenu par la platine supérieure 10 tandis que le fond de boîte 90' se loge dans l'ouverture 110 de la platine inférieure 11. Le disque moteur 6 est alors entraîné sur un angle de 40° afin d'acheminer la boîte 9', en position ouverte, vers la station de remplissage. Cette rotation est figurée par la flèche $F_1$ à la figure 1 ; elle se fait dans le sens des aiguilles d'une montre, c'est-à-dire en sens contraire de celui des carrousels. Une paire d'ouvertures 100, 110 vient donc alors se positionner sous l'ouverture 30a, et l'opération peut recommencer pour le prélèvement de la dernière boîte de la pile correspondant à la colonne suivante se trouvant dans le carroussel 2a ; à cet effet ce dernier est tourné d'un angle de 20° toujours dans le même sens.

Les boîtes prélevées arrivent donc successivement au poste de remplissage, ce mouvement étant figuré par la flèche j à la figure 7.

Pendant ce déplacement, selon une caractéristique importante de l'invention, les fonds de boîtes 90 se trouvent en appui sur la semelle 55, c'est-à-dire sur un plan réfrigéré. Cette réfrigération des boîtes, avant et après remplissage, va favoriser le refroidissement de la gélose et donc en accélérer le durcissement.

Lorsqu'une boîte à remplir est arrivée au niveau de la buse 70, le microprocesseur commande la mise en fonctionnement de la pompe 75, laquelle réalise l'envoi à la buse d'une dose de gélose déterminée 95, qui est injectée dans le fond de boîte 90.

Après remplissage, toujours transportées par le plateau tournant 1, les boîtes arrivent successivement à l'état ouvert au-dessus du piston 4b, lequel se trouve dans sa position basse (face 48 à fleur de la semelle 55. A ce stade, en raison du rôle de réfrigération de la semelle d'appui 55, la gélose s'est pratiquement solidifiée. La came 42b va provoquer alors le stockage de la boîte pleine à l'intérieur du carrousel récepteur 2b, ceci par un mode opératoire similaire mais inversé par rapport à l'opération de prélèvement des boîtes vides. Pour cela, la came tournante 42 réalise tout d'abord la remontée du piston 4b à un niveau intermédiaire correspondant à un retrait d'une hauteur d'une demi-boîte par rapport à la plaque 3. Au cours de ce soulèvement, le piston 4 amène le fond 90 à l'intérieur du couvercle 91 et réalise par conséquent la fermeture automatique de la boîte. Le piston étant dans sa position intermédiaire, le carrousel récepteur 2b est tourné de manière à amener une colonne de boîtes pleines en appui sur la boîte qui se trouve en attente ; le piston 4b est alors complètement soulevé et est introduit dans le bas de la pile de boîte en soulevant l'ensemble des autres boîtes ; le carrousel 2b est alors tourné de 20° de manière à amener l'ensemble de la pile à cheval entre l'ouverture 30b et la surface d'appui constituée par la plaque 3, dans l'attente de la boîte suivante.

Il faut remarquer que les fonds de boîtes présentent souvent une certaine concavité, délimitée par un rebord en saillie 900 ; durant le passage de la colonne de boîtes pleines du piston sur la plaque 3, le pan incliné 400b évite que ce rebord 900 n'accroche contre le piston (voir figure 9) ce qui aurait pour effet de faire basculer la boîte, laquelle risquerait alors de s'accrocher contre le bord de l'ouverture 30b.

Aux figures 8 et 9, les mouvements de soulèvement du piston et de déplacement de la colonne de boîtes pleines ont été figurés respectivement par les flèches k et l.

A la figure 7, les flèches R représentent le flux d'air réfrigérant les ailettes du système de refroidissement 8 à effet Peltier.

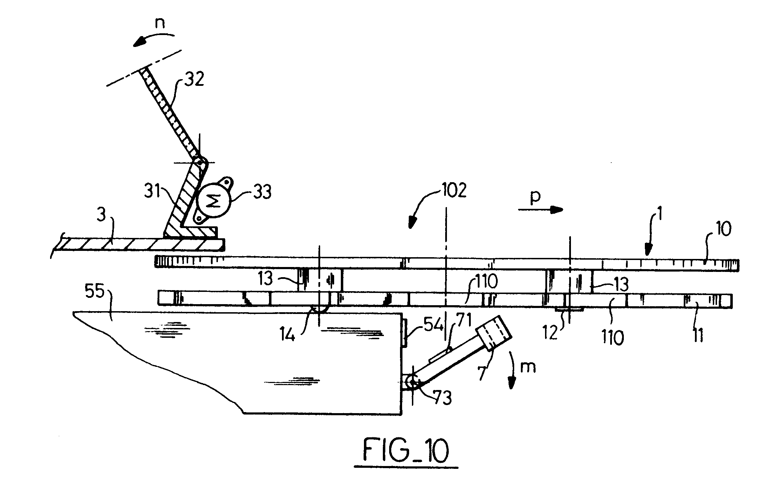

La figure 10 montre de quelle manière il est très facile d'enlever le plateau 1 notamment pour nettoyer ce plateau et la semelle d'appui 55 dans le cas où de la gélose s'est déversée inopinément dans le dispositif. Pour cela, le capot 32 est tout d'abord relevé (flèche n) ; la buse 70 ayant été enlevée, le levier 7 est basculé vers le bas (flèche m), ce qui libère la face avant 102 du dispositif. En exerçant sur le plateau 1 un effort horizontal p vers l'avant, suffisant pour vaincre les forces d'attraction magnétique de l'accouplement 61-12, on provoque le déplacement et l'extraction du plateau 1. Au cours de ce déplacement, les billes 14 roulent sur la semelle 55, ce qui facilite encore l'opération. Après nettoyage il suffit d'effectuer les mêmes mouvements en sens contraire et le plateau se trouve automatiquement en bonne position.

Le microprocesseur est programmé de telle manière que lorsque le détecteur 72 détecte l'absence d'un fond de boîtes le microprocesseur ne commande pas la mise en route de la pompe 75. Ceci se produit en particulier lorsqu'une boîte s'est trouvée placée à l'envers dans le carrousel 2a ; en effet, l'ensemble de la boîte est alors retenue par la platine supérieure 10. Du fait que la pompe n'est pas mise en fonctionnement, il ne se produit pas de distribution de gélose intempestive, et l'opération continue normalement, sans interruption pour les autres boîtes. La boîte placée à l'envers est stockée normalement, toujours à l'envers, avec les boîtes pleines dans le carrousel récepteur 4b.

A titre indicatif, les vitesses d'avancement sont choisies de façon à obtenir une cadence de production de l'ordre d'une boîte toutes les six secondes. Les modules à effet Peltier sont choisis, pour une utilisation à température ambiante, pour amener la température du plateau réfrigéré à une valeur de l'ordre de 7 à 10° C. Dans ces conditions, la gélose est pratiquement dure lorsque la boîte remplie arrive au-dessus du piston 4b, de sorte que les mouvements de la boîte au cours de son stockage ne détruise pas ou pratiquement pas la planéité de la surface de la gélose.

Il peut être prévu d'équiper le bâti du dispositif d'un niveau à bulle permettant de règler parfaitement l'horizontalité de la semelle 55, ceci afin de garantir la régularité d'épaisseur de la couche de produit.

Du fait que les boîtes stockées sont déjà refroidies, il est possible de les utiliser immédiatement.

## Revendications

1. Dispositif pour remplir des boîtes de Pétri, ces boîtes ayant une forme circulaire, comprenant un fond sur lequel s'emboîte librement un couvercle de diamètre légèrement supérieur à celui du fond, du type comprenant un plateau de transfert tournant (1) apte à acheminer successivement et pas-à-pas des boîtes ouvertes (9) - couvercle (91) maintenu écarté au-dessus du fond (90) - d'une station distributrice de boîtes vides à une station de remplissage, puis de cette dernière à une station réceptrice de boîtes remplies, ce plateau (1) étant composé de deux platines horizontales circulaires (10, 11) coaxiales et superposées percées chacune d'une série d'ouvertures circulaires régulièrement réparties à leur périphérie et en correspondance l'une avec l'autre, les ouvertures (100) de la platine supérieure (10) ayant un diamètre légèrement plus petit que celui des couvercles (91) mais légèrement plus grand que celui des fonds (90) de sorte qu'elles autorisent le passage de ces derniers en retenant les couvercles, caractérisé en ce que :

    – d'une part, les ouvertures (110) de la platine inférieure (11) ont un diamètre légèrement plus grand que celui des fonds (90) de telle sorte qu'elles autorisent également le passage de ces derniers ;

    – d'autre part, ledit plateau de transfert (1) repose, par sa platine inférieure (11) sur une semelle fixe (55), plane et horizontale, de telle manière que durant leur transfert au moyen de ladite platine inférieure (11), les fonds de boîte (90) se trouvent en appui sur ladite semelle (55), l'entraînement du plateau (1) en rotation étant réalisé par des moyens moteurs (63, 62, 6) situés sous cette semelle (55), par l'intermédiaire d'un accouplement magnétique (61, 12).

2. Dispositif selon la revendication 1, caractérisé en ce que ladite semelle est réfrigérée, par exemple au moyen de modules de refroidissement à effet Peltier (8).

3. Dispositif selon l'une des revendications 1 ou 2, caractérisé en ce que lesdites stations distributrice et réceptrice comprennent des moyens de stockage distincts constitués par des carrousels identiques (2a, 2b) dans lesquels les boîtes (9) sont empilées en colonnes, la rotation pas-à-pas de ces carrousels se faisant en synchronisme avec celle dudit plateau de transfert (1) de sorte que les boîtes vides quittent une à une le carrousel distributeur (2a) en s'échappant successivement du bas de colonnes voisines tandis que les boîtes remplies sont introduites une à une dans le carrousel récepteur (2b) par la base de colonnes voisines.

4. Dispositif selon la revendication 3, caractérisé en ce que les colonnes de boîtes contenues dans lesdits carrousels (2a, 2b) reposent sur une plaque horizontale (3) recouvrant ledit plateau de transfert (1), cette plaque étant traversée, à chacune des stations distributrice et réceptrice, d'une ouverture (30a, 30b) qui autorise le passage des boîtes du carrousel distributeur (2a) au plateau (1), et de ce dernier au carrousel récepteur (2b).

5. Dispositif selon la revendication 4, caractérisé en ce que les moyens d'entraînement en rotation desdits carrousels (2a, 2b) impriment à ceux-ci, pour chaque boîte, deux demi-courses successives correspondant à la moitié de l'écart angulaire entre deux colonnes voisines.

6. Dispositif selon la revendication 5, caractérisé en ce qu'il est pourvu de deux pistons de transfert (4a, 4b) situés l'un à la station distributrice, et l'autre à la station réceptrice et dont le diamètre est sensiblement plus petit que celui des boîtes, des moyens - tels que des cames rotatives (42) - étant prévus pour déplacer verticalement ces pistons (4a, 4b) dans l'axe de l'ouverture associée (30a, 30b).

7. Dispositif selon la revendication 6, caractérisé en ce que lesdits moyens (42) permettent d'amener successivement le piston (4a, 4b) dans chacune des positions suivantes :

    a) une position dite haute, dans laquelle sa face supérieure (48) se trouve sensiblement à fleur de ladite plaque horizontale (3) ;

    b) une position dite intermédiaire, dans laquelle sa face supérieure (48) se trouve à un niveau inférieur de celui de ladite plaque horizontale (3), d'une hauteur correspondant sensiblement à celle d'une boîte (9) ;

    c) une position dite basse, dans laquelle sa face supérieure (48) se trouve sensiblement à fleur de ladite semelle (55).

8. Dispositif selon l'une des revendications 6 ou 7, caractérisé en ce que la face supérieure (48) du piston (4a, 4b) présente un pan biseauté (400) et/ou un chanfrein (401).

9. Dispositif selon l'une des revendications 1 à 8, caractérisé en ce que l'entraînement du plateau de transfert (1) est réalisé au moyen d'un disque moteur horizontal (6) disposé juste en-dessous de la semelle (55), à l'aplomb du plateau (1), ce disque (6) et la platine inférieure (11) étant pourvus chacun d'une série d'aimants (61, 12) régulièrement répartis à leur périphérie en vis-à-vis les uns des autres.

10. Dispositif selon l'une des revendications 1 à 8, caractérisé en ce que ladite platine inférieure (11) prend appui sur la semelle (55) par l'intermédiaire de moyens antifriction tels que des billes (14) et/ou des roulements à billes.

11. Dispositif selon l'une des revendications 1 à 10, caractérisé en ce que ladite station de remplissage comprend une buse d'injection (70) de produit tel que de la gélose (95), qui est alimentée par une pompe, et est normalement positionnée horizontalement entre les deux platines (10, 11) du plateau de transfert, et que cette buse (70) est portée par un levier, (7) pouvant être escamoté par basculement pour dégager l'accès de la semelle (55) et autoriser l'enlèvement dudit plateau (1).

12. Dispositif selon l'une des revendications 1 à 11, caractérisé en ce que ladite platine inférieure (11) est échancrée vers l'extérieur au niveau de chaque ouverture (110), de manière à permettre une détection de la présence d'un fond de boîte (90) dans cette ouverture au moyen d'un détecteur opto-électronique (72) situé à la station de remplissage.

13. Dispositif selon l'une des revendications 1 à 12, caractérisé en ce qu'il est équipé d'un dispositif germicide comprenant un tube de rayonnement ultraviolet (33), auquel sont exposées les boîtes (9) au cours de leur passage à la station de remplissage.

## Patentansprüche

1. Vorrichtung zum Füllen von Petrischalen, wobei diese Schalen eine kreisförmige Form besitzen, mit einem Boden, auf dem sich frei ein Deckel mit einem Durchmesser, der geringfügig größer ist als der des Bodens, aufstülpt, der Art, die eine drehende Transportplatte (1) umfaßt, die geeignet ist, sukzessive und Schritt für Schritt offene Schalen (9) - wobei ein Deckel (91) von dem Boden (90) entfernt darüber gehalten wird - von einer Verteilerstation für leere Schalen zur einer Abfüllstation und dann von letzterer zu einer Aufnahmestation für gefüllte Schalen zu transportieren, wobei diese Platte aus zwei horizontalen, kreisförmigen kleine Platten (10, 11) zusammengesetzt ist, die koaxial und übereinander angeordnet sind und jeweils von einer Reihe von kreisförmigen Öffnungen durchbohrt sind, die regelmäßig an ihrem Umfang verteilt sind und jeweils einander entsprechen, wobei die Öffnungen (100) in der oberen kleine Platte (10) einen etwas geringeren Durchmesser als den der Deckel (91) aber etwas größer als den der Böden (90) besitzen, so daß sie den Durchgang der letzteren erlauben, wobei die Deckel zurückgehalten werden, dadurch gekennzeichnet, daß:

– auf der einen Seite die Öffnungen (110) der unteren kleinen Platte (11) einen etwas größeren Durchmesser als den der Böden (90) besitzen, so daß sie ebenfalls den Durchgang der letzteren erlauben;

– auf der anderen Seite die Transportplatte (1) über ihre untere kleine Platte (11) auf einem festen, ebenen und horizontalen Sitz (55) ruht, so daß während des Transports mittels der unteren kleinen Platte (11) die Schalenböden (90) sich aufstützend auf dem Sitz (55) befinden, wobei die Mitnahme der sich drehenden Platte (1) durch Motorvorrichtungen (63, 62, 6), die sich unter diesem Sitz (55) befinden, über eine magnetische Kupplung (61, 12) erfolgt.

2. Vorrichtung nach Anspruch 1, dadurch gekennzeichnet, daß der Sitz abgekühlt wird, zum Beispiel mittels Kühlmodulen nach dem Peltiereffekt (8).

3. Vorrichtung nach einem der Ansprüche 1 oder 2, dadurch gekennzeichnet, daß die Verteiler- und Aufnahmestationen getrennte Speichervorrichtungen umfassen, die aus identischen Karussells (2a, 2b) bestehen, in denen die Schalen (9) in Reihen gestapelt sind, wobei die schrittweise Drehung dieser Karussells synchron mit der der Transportplatte (1) abläuft, so daß die leeren Schalen eine nach der anderen das Verteilerkarrousell (2a) verlassen, in dem sie sukzessive von der Unterseite benachbarter Reihen entweichen, während die gefüllten Schalen eine nach der anderen in das Aufnahmekarrusell (2b) über die Unterseite benachbarter Reihen aufgenommen werden.

4. Vorrichtung nach Anspruch 3, dadurch gekennzeichnet, daß die in den Karrusells (2a, 2b) enthaltenen Schalenreihen auf einer horizontalen Platte (3) aufliegen, die die Transportplatte (1) bedeckt, wobei diese Platte sowohl in der Verteiler- als auch der Aufnahmestation von einer Öffnung (30a, 30b) durchstoßen wird, die den Durchgang der Schalen von dem Verteilerkarrusell (2a) zur Platte (1) und von letzterer zum Aufnahmekarrusell (2b) erlaubt.

5. Vorrichtung nach Anspruch 4, dadurch gekennzeichnet, daß die Vorrichtungen zum In-Drehung-Versetzen der Karrusells (2a, 2b) diesen für jede Schale zwei aufeinander folgende Halbdrehungen aufprägen, die der Hälfte des Winkelabstands zwischen zwei benachbarten Reihen entspricht.

6. Vorrichtung nach Anspruch 5, dadurch gekennzeichnet, daß sie mit zwei Transportkolben (4a, 4b) versehen ist, von denen der eine an der Verteilerstation und der andere an der Aufnahmestation angeordnet ist und deren Durchmesser deutlich kleiner ist als der der Schalen, wobei Vorrichtungen wie etwa sich drehende Nocken (42) - vorgesehen sind, um diese Kolben (4a, 4b) vertikal entlang der Achse der entsprechenden Öffnung (30a, 30b) zu bewegen.

7. Vorrichtung nach Anspruch 6, dadurch gekennzeichnet, daß diese Vorrichtungen (42) erlauben, den Kolben (4a, 4b) der Reihe nach in jede der folgenden Positionen zu bewegen:

a) eine sogenannte hohe Position, in der sich die obere Seite (48) ungefähr fluchtend mit der horizontalen Platte (3) befindet;
b) eine sogenannte Zwischenposition, in der sich die obere Seite (48) auf einem Niveau unterhalb der horizontalen Platte (3) auf einer Höhe befindet, die ungefähr der einer Schale (9) entspricht;
c) eine sogenannte tiefe Position, in der sich die obere Seite (48) ungefähr fluchtend mit dem Sitz (55) befindet.

8. Vorrichtung nach einem der Ansprüche 6 oder 7, dadurch gekennzeichnet, daß die obere Seite (48) des Kolbens (4a, 4b) ein abgeschrägtes Feld (400) und(oder eine Abfasung (401) aufweist.

9. Vorrichtung nach einem der Ansprüche 1 bis 8, dadurch gekennzeichnet, daß die Mitnahme der Transportplatte (1) mittels einer horizontalen Motorscheibe (6) erfolgt, die genau unter dem Sitz (55) in der Fallinie der Platte (1) angeordnet ist, wobei diese Scheibe (6) und die untere kleine Platte (11) jeweils mit einer Reihe von Magneten (61, 62) versehen sind, die regelmäßig auf ihrem Umfang verteilt und einander gegenüber angeordnet sind.

10. Vorrichtung nach einem der Ansprüche 1 bis 8, dadurch gekennzeichnet, daß sich die untere kleine Platte (11) über Antireibungsvorrichtungen, wie etwa über Kugeln (14) und/oder Kugellager, auf den Sitz (55) stützt.

11. Vorrichtung nach einem der Ansprüche 1 bis 10, dadurch gekennzeichnet, daß die Abfüllstation eine Injektionsdüse (70) für das Produkt, wie etwa für Nährbodenmaterial (95), umfaßt, die über eine Pumpe versorgt wird und sich normalerweise horizontal zwischen den beiden kleinen Platten (10, 11) der Transportplatte befindet, und daß diese Düse (70) von einem Hebel (7) gehalten wird, der durch Kippen entfernt werden kann, um den Zugang zu dem Sitz (55) freizumachen und das Entfernen der Platte (1) zu ermöglichen.

12. Vorrichtung nach einem der Ansprüche 1 bis 11, dadurch gekennzeichnet, daß die untere kleine Platte (11) auf der Höhe jeder Öffnung (110) nach außen geschweift ist, um eine Detektion eines Schalenbodens (90) in dieser Öffnung mittels eines sich an der Abfüllstation befindlichen optoelektronischen Detektors (72) zu ermöglichen.

13. Vorrichtung nach einem der Ansprüche 1 bis 12, dadurch gekennzeichnet, daß sie mit einer keimtötenden Vorrichtung ausgerüstet ist, die ein Röhre für ultraviolette Strahlung (33) umfaßt, der die Schalen (9) während ihres Übergangs zur Abfüllstation ausgesetzt werden.

**Claims**

1. Device for filling petri dishes, said dishes being circular in shape, comprising a bottom onto which a lid of slightly larger diameter than the bottom is fitted freely, the apparatus being of the type comprising a transfer turntable (1) suitable for conveying open dishes (9) having their lids (91) held up at a distance above their bottoms (90) in succession and step by step from an empty dish dispensing station to a dish-filling station, and then from the dish-filling station to a filled dish receiving station, said turntable (1) comprising two superposed coaxial circular horizontal plates (10, 11) each pierced by a series of circular openings regularly distributed around its periphery and in register with the openings in the other disk, the diameter of the openings (100) in the top disk (10) being slightly smaller than the diameter of the lids (91) but slightly larger than the diameter of the bottoms (90), thereby allowing the bottoms to pass therethrough while retaining the lids, characterized in that :
– firstly the openings (110) in the bottom plate (11) are slightly larger in diameter than the bottoms (90) so that they too allow the bottoms to pass therethrough; and
– secondly the said transfer turntable (1) rests via its bottom plate (11) on a fixed sole plate (55) which is plane and horizontal, such that the dish bottoms (90) rest on said sole plate (55) while they are being transferred by means of said bottom plate (11), the turntable (1) being rotated by drive means (63, 62, 6) situated beneath the sole plate (55) and acting via magnetic coupling (61, 12).

2. Device according to claim 1, characterized in that the said sole plate is refrigerated, e.g. by means of Peltier effect cooling modules (8).

3. Device according to claim 1 or 2, characterized in that the said dish-distributing and receiving stations comprise distinct storage means constituted by identical carousels (2a, 2b) in which the dishes (9) are stacked in columns, stepwise rotation of the carousels taking place synchronously with rotation of the said transfer turntable (1) in such a manner that the empty dishes leave the dispensing carousel (2a) one by one by escaping in succession from the bottoms of adjacent columns, while filled dishes are inserted one by one into the receiving carousel (2b) via the bases of adjacent columns .

4. Device according to claim 3, characterized in that the columns of dishes contained in said carousels (2a, 2b) rest on a horizontal slab (3) overlying said transfer turntable (1), said slab being pierced at each of the distributing and receiving stations by respective openings (30a, 30b) allowing dishes to pass from the dispensing carousel (2a) to the turntable (1) and from the turntable to the receiving carousel (2b).

5. Device according to claim 4, characterized in that the means for rotating the said carousels (2a, 2b) impart two successive half-strokes to each carousel for each dish, each half-stroke corresponding to one half of the angular spacing between two adjacent columns.

6. Device according to claim 5, characterized in that two transfer pistons (4a, 4b) are provided, one situated at the dispensing station and the other at the receiving station, the pistons being slightly smaller in diameter than the dishes, and means such as rotary cams (42) are provided for displacing the pistons (4a, 4b) vertically on the axes of the associated openings (30a, 30b).

7. Device according to claim 6, characterized in that the said means (42) serve to bring each piston (4a, 4b) into each of the following positions in succession:

a) a high position in which the top face (48) of the piston lies substantially flush with said horizontal slab (3);

b) an intermediate position in which the top face (48) lies below the lever of the horizontal slab (3) by an amount corresponding substantially to the height of one dish (9); and

c) a low position in rich the top face (48) lies substantially flush with said sole plate (55).

8. Device according to claim 6 or 7, characterized in that the top face (48) of each piston (4a, 4b) has a sloping flat (400) and/or a chamfer (401).

9. Device according to any one of claims 1 to 8, characterized in that the transfer turntable (1) is driven by means of a horizontal drive disk (6) disposed immediately beneath the sole plate (55) vertically below the turntable (1), said drive disk (6) and the bottom plate (11) each being provided with a series of magnets (61, 12) regularly distributed around their respective peripheries and facing one another.

10. Device according to any one of claims 1 to 8, characterized in that the said bottom plate (11) bears against the sole plate (55) via antifriction means such as balls (14) and/or ball bearings.

11. Device according to any one of claims 1 to 10, characterized in that said filling station comprises a nozzle (70) for injecting a substance such as gelose (95), which nozzle is fed by a pump and is normally positioned horizontally between the two plates (10, 11) of the transfer turntable, with the nozzle (70) being carried by a lever (7) capable of being retracted by being tilted so as to disengage the access to the sole plate (55), thereby enabling said turntable (1) to be removed.

12. Device according to any one of claims 1 to 11, characterized in that said bottom plate (11) is notched to the outside at each of its openings (110), thereby enabling the presence of a dish bottom (90) in each opening to be detected by means of an opto-electronic detector (72) situated at the filling station.

13. Device according to any one of claims 1 to 12, characterized in that it is provided with a germicidal device comprising an ultraviolet radiating tube (33) to which the dishes (9) are exposed as they pass to the filling station.

FIG_1

FIG_2

EP 0 385 902 B1

FIG_3

FIG_4

FIG_4a

FIG_5

FIG_5a

FIG_6

FIG_7

FIG_8

FIG_9

FIG_10